# EUROPEAN PATENT SPECIFICATION

(11) **EP 2 539 461 B1**
(45) Date of publication and mention of the grant of the patent: **07.01.2015**
(21) Application number: 11708400.4
(22) Date of filing: 24.02.2011
(51) Int. Cl.: C12Q 1/68

(54) **Genetic marker for the diagnosis of dementia with lewy bodies**
Genetischer Marker zur Diagnose von Lewy-Körper-Demenz
Marqueur génétique pour le diagnostic de la démence à corps de Lewy

(30) Priority: 24.02.2010 EP 10382042
(43) Date of publication of application: 02.01.2013
(73) Proprietor: Universitat Autònoma De Barcelona, 08193 Cerdanyola del Vallès (ES); Fundació Institut d'Investigació en Ciències de la Salut Germans Trias i Pujol, 08916 Badalona, Barcelona (ES)
(72) Inventor: BEYER, Katrin, 08916 Badalona (ES); DOMINGO SÀBAT, Montserrat, 08912 Badalona (ES); ARIZA FERNÁNDEZ, Aurelio, 08193 Cerdanyola del Vallès (ES)
(74) Representative: Durán-Corretjer, S.L.P.
(86) International application number: PCT/EP2011/000903
(87) International publication number: WO 2011/104023

(56) References cited:
- WO-A2-98/27226
- PRIMO-PARMO S L ET AL: "Characterization of 12 silent alleles of the human butyrylcholinesterase (BCHE) gene", AMERICAN JOURNAL OF HUMAN GENETICS, vol. 58, no. 1, 1996, pages 52-64, XP002577524, ISSN: 0002-9297
- SINGLETON A ET AL: "Butyrylcholinesterase K: an association with dementia with Lewy bodies", LANCET THE, LANCET LIMITED. LONDON, GB LNKD- DOI:10.1016/S0140-6736(05)78788-2, vol. 351, no. 9118, 13 June 1998 (1998-06-13), page 1818, XP004834745, ISSN: 0140-6736 cited in the application
- MAETZLER W ET AL: "No differences of butyrylcholinesterase protein activity and allele frequency in Lewy body diseases", NEUROBIOLOGY OF DISEASE, BLACKWELL SCIENTIFIC PUBLICATIONS, OXFORD, GB LNKD- DOI:10.1016/J.NBD.2009.05.014, vol. 35, no. 2, 1 August 2009 (2009-08-01) , pages 296-301, XP026265790, ISSN: 0969-9961 [retrieved on 2009-05-27] cited in the application
- LANE ROGER ET AL: "BuChE-K and APOE epsilon4 allele frequencies in Lewy body dementias, and influence of genotype and hyperhomocysteinemia on cognitive decline.", MOVEMENT DISORDERS : OFFICIAL JOURNAL OF THE MOVEMENT DISORDER SOCIETY 15 FEB 2009 LNKD- PUBMED:19006190, vol. 24, no. 3, 15 February 2009 (2009-02-15), pages 392-400, XP002577525, ISSN: 1531-8257 cited in the application

## Description

The present invention relates to the field of medicine, and particularly to neurodegenerative disorders. It specifically relates to markers for the diagnosis of dementia with Lewy bodies.

### BACKGROUND ART

Lewy body diseases comprise a group of disorders characterized by the presence of proteinaceous neuronal inclusions called Lewy bodies (LB). Clinically, two disorders can be distinguished: Parkinson disease (PD) and dementia with Lewy bodies (DLB). Whereas PD is the most common progressive movement disorder in the elderly, DLB is the second most frequent cause of dementia after Alzheimer disease (AD). While widespread distribution of LB in virtually every brain area is a typical feature of DLB, the *substancia nigra* is the most affected in PD.

When first described, DLB was thought to be an infrequent disorder, but over the last years intense investigation has revealed that it accounts for 10-15% of autopsied cases. Main DLB symptoms include fluctuating cognitive impairment, recurrent visual hallucinations and Parkinsonism, but nevertheless, many AD overlapping symptoms lead to a frequent misdiagnosis of DLB. Since AD and DLB patients may differ in terms of response to medication and prognosis, it is important to improve accuracy in diagnosing DLB.

To achieve a better clinical distinction between DLB and AD, various longitudinal and comparative studies have been carried out during the last years. Patients with only Lewy body (LB) pathology show relatively less severe impairments but more pronounced deterioration of executive function and attention than patients with only AD or mixed AD/LB pathology. Moreover, DLB patients exhibit a slower decline of recognition memory but have more psychiatric symptoms than patients with AD, where this kind of symptomathology is observed at later disease stages. Finally, the presence of visual hallucinations in early-stage dementia has been shown to be most specific for DLB. It is noteworthy to mention that although a high specificity (ranging from 90 to 99% in different studies) of clinical diagnosis is achieved, its sensitivity remains relatively low (18-83%). Accordingly, the first consensus guidelines established in 1996 for the clinical diagnosis of probable and possible DLB have been revised to improve the sensitivity for DLB diagnosis, but nevertheless, many AD overlapping symptoms lead to a frequent misdiagnosis of DLB between 40-80% of the cases.

The main cause of low diagnostic sensitivity for DLB comes from the elevated percentage of cases that show in addition to LB related pathology AD characteristic changes. To assess this type of combined pathology, the third DLB consortium proposed a model to place AD-related pathology into the context of LB pathology. The higher the stage of AD-type pathology the lower is the sensitivity to achieve a correct diagnosis of DLB. Accordingly, a recent report confirmed that the misdiagnosis of DLB increases with increasing AD associated pathology, but even so, only around 52% of patients had received the correct diagnosis of DLB at low AD-pathology stages.

The treatment of DLB is symptomatic and is based on a limited number of clinical trials and extension of results from trials in AD. At the moment AD treatment consists of using cholinesterase inhibitors to improve the effectiveness of acetylcholine either by increasing the levels in the brain or by strengthening the way nerve cells to respond to it. Moreover, neuroleptic drugs are used to diminish psychotic symptoms normally present during the disease course. On the contrary, for treating DLB the use of neuroleptics may cause adverse reaction in about 50% of DLB patients and may cause death.

Thus, the ability to differentially diagnose between AD and DLB will be a major advantage not only for the individual patient being treated, but also with respect to the economic strains of public health systems. However, at present, precise differentiation of AD and DLB is only possible by post-mortem analysis of brain tissue.

Nowadays, diagnosis of DLB is based on clinical evaluation of symptoms and traits, following the guidelines established by the Consortium on DLB International Workshop (I.G. McKeith, "Consensus guidelines for the clinical and pathologic diagnosis of dementia with Lewy bodies (DLB): report of the Consortium on DLB International Workshop", J. Alzheimer's Dis. 2006, vol. 9, pp. 417-23), but as explained above, it leads to misdiagnosis of DLB. Image methods like positron tomography (PET) and single photon emission computer tomography (SPECT) are available, but their sensitivity is not very high and they are very expensive for a routine clinical use. An early unequivocal diagnosis would give a therapeutic margin to reduce or stop the disease progression.

There have been some attempts in trying to find genetic markers to precisely identify a patient with DLB. A genetic test would be a very useful tool and easy to perform in the daily clinical practice in the pre-mortem diagnosis of DLB. In this sense, some proteins and genes studied in order to find a relationship with DLB are alpha-7 nicotinic acetylcholine receptor subunit, osteopontin, nitric oxide synthase, ubiquitin carboxy-terminal hydrolase L1 gene, BDNF gene, or beta-synuclein gene. Many of them have been studied in brain samples at an experimental level and they are not useful in real clinical diagnosis because of the difficulties to obtain a patient brain biopsy.

Butyrylcholinesterase (BChE) is a glycoprotein enzyme synthesized in the liver. In the human brain it is found principally in glia, particularly in cortical and subcortical structures, but it is also found in neurons above all, those implicated in cognitive functions. In AD patients BChE is found in amyloid plaques, as well as, in neurofibrillary tangles. This enzyme acts as a detoxification enzyme of organophosphorus and carbamate compounds and hydrolyzes succinylcholine, aspirin and cocaine. BChE function in the human brain is not well known, but it is known that hydrolyzes acetylcholine (ACh) when acetylcholinesterase (AChE) is reduced or absent. It is a marker for determining apnea susceptibility. Up to the moment 65 variants have been identified in BChE gene which is located in chromosome 3 (3q26.1-q26.2) (cf. F. Parmo-Folloni et al., "Two new mutations of the human BCHE gene (IVS3-14T>C and L574fsX576)" Chemico-Biological Interactions 2008, vol. 175, pp. 135-7).

The presence of mutation A539T in exon 4 of BChE gene is named K variant in honor of Werner Kalow. The K-variant is associated with a DNA transition from guanine to adenine at nucleotide 1615 in the mRNA corresponding to position 68974 in the DNA sequence (NCBI Accession Number NG_009031), which causes an amino acid change from alanine 539 to threonine.

The K-variant is situated at the C-terminal of the protein, responsible for its tetramerization on one hand, and for the attenuation of beta-amyloid fibril formation, on the other. In serum the BChE K variant is responsible for a one third reduction of serum BChE activity levels. Although main BChE functions in brain remain unknown, the K-variant seems to diminish the rate of attenuation of beta-amyloid fibril formation, accelerating AD progression. On the contrary, tau protein is less phosphorylated in AD patients that carry at least one K-allele, representing a protective mechanism for AD.

Many studies have investigated a possible association between BChE gene, specially the BChE K variant, and AD. Co-occurrence of the epsilon 4 allele of the apolipoprotein E gene (ApoE4), the major known genetic risk factor for AD, and BChE gene variants have been discussed to influence AD pathology. Some reports show an increased risk for AD in subjects with a combination of BChE wild type and ApoE4 genotype. Others found that the combination of BChE K and the ApoE4 increased the risk for AD. The progression of cognitive decline in AD has been shown to be influenced by the BChE genotype. However, there is not a definitive conclusion about the role of BChE K variant as neither a risk factor nor a progression marker for AD.

The possible association of BChE K genotype and DLB has also been studied. Singleton et al. (A.B. Singleton et al., "Butyrylcholinesterase K: an association with dementia with Lewy bodies", Lancet 1998, vol. 351, pp. 1818) reported an increased frequency of homozygous BChE K carriers in DLB compared to controls. A recent study found increased BChE K and ApoE4 frequencies in DLB patients compared to PDD patients (R. Lane et al., "BuChE-K and APOE epsilon4 allele frequencies in Lewy body dementias, and influence of genotype and hyperhomocysteinemia on cognitive decline", Mov. Disord. 2009, vol. 24, pp. 392-400). Based on the hypothesis that a higher percentage of DLB than PDD subjects have additional AD-type pathology, and additional AD type pathology leads to more rapid cognitive decline, the authors concluded that this genotype may be important in dementia onset and progression in LBD. However, a recent study shows that there is not a significant association between the BChE K variant and the demented DLB phenotype (cf. W. Maetzler et al., "No differences of butyrylcholinesterase protein activity and allele frequency in Lewy body diseases" Neurobiol. Dis. 2009, vol. 35, pp. 296-301).

Therefore, there is the need of providing means for an accurate identification of a patient suffering from dementia of Lewy bodies, and distinguishing from Alzheimer disease, to be used in the common clinical practice.

### SUMMARY OF THE INVENTION

The inventors have found specific alterations in BChE gene which allow determining whether a patient suffers from dementia with Lewy bodies, and distinguishing it from Alzheimer disease.

There are documents in the state of the art that intend to find an association between BChE K variant and DLB, but as explained below, recent studies consider that there is no significant association (cf. W. Maetzler et al., *supra*). Surprisingly, the inventors of the present invention have found that specific information for diagnosis of DLB is obtained with genotype of K variant in co-occurrence with the genotype of a so far unknown alteration in BChE gene, a poly-thymine region in positions 4780 to 4786. Determining these two genotypes is therefore useful to distinguish DLB from AD, these two genotypes constituting a specific genetic marker for DLB.

The inventors have further observed that a combination of genotypes gives rise to identify a group of patients suffering from DLB, and distinguishing from AD. This combination is formed by the genotypes of the polymorphic sites at positions 3687, 4206, and 4443, the polythymine region at positions 4780 to 4786 in NCBI Accession Number NG_009031 (i.e. positions 3687, 4206, 4443 and 4780-4786 respectively in SEQ ID NO: 1), and the polymorphic site at position 68974 in NCBI Accession Number NG_009031 (i.e. position 934 in SEQ ID NO: 28).

Positions of the alterations in BChE nucleotide sequence are given from the nucleotide sequence of NCBI Accession Number NG_009031 which corresponds to the promoter and the gene. This sequence was published on 31 January 2010.

The polymorphic sites 3687, 4206, 4443 and the polythymine region at positions 4780 to 4786 are in the promoter region. For these sites, reference is made also to the SEQ ID NO: 1, which corresponds to the sequence from nucleotide 1 to nucleotide 5040 of the complete sequence of BChE at NCBI. A possible numbering of the nucleotides sometimes used takes the transcription start as position 1 and consequently, the nucleotides upstream this position as negative positions. Transcription start position 1 corresponds to position 5001 in NG_0090031. The correspondence between the numbering used in this description and the "negative" one, is given herein:
A3687G corresponds to A-1314G
A4206G corresponds to A-795G
C4443T corresponds to C-558T
polythymine from 4780 to 4786 corresponds to -215 to -221

The polymorphic site at position 68974 is in the codifying region of NG_009031. The region from position 68041 to 7020 of NG_009031 is included as SEQ ID NO: 28. Taking this region alone, the nucleotides are renumbered, so consequently, the position 68974 in the complete gene sequence becomes the position 934 in SEQ ID NO: 28. This polymorphism is associated to the change of amino acid in exon 4 of BChE resulting in the K variant. The position also used in the literature for this polymorphism is 1615 due to a different sequence numbering (with reference to the mRNA sequence which codifies for the mature BChE protein, without the signal peptide).

As described in the examples below, no specific association has been found between each of the five alterations in BChE gene independently evaluated and DLB; but surprisingly, these alterations in combination give specific information for DLB.

Accordingly, an aspect of the invention provides an *in vitro* method for the diagnosis of DLB comprising determining in a biological sample from a subject, the genotype of the following alterations in butyrylcholinesterase (BChE) gene: the polymorphic site at position 68974 and the poly-thymine region at positions 4780 to 4786.

In a particular embodiment, the method further comprises determining the genotype of the following alterations in BChE gene : the polymorphic sites at position 3687, 4206, and 4443.

As it is shown in the examples below, post mortem samples of AD (n=26), pure DLB (n=12), common DLB (n=24) and controls (n=23) were analyzed, as well as clinically diagnosed samples obtained from 223 AD and 160 control subjects. As a result, three types of genetic markers are described. First, the co-occurrence of specific changes at two polymorphic sites is characterized by the presence of one allele with 7 thymines at positions 4780 to 4786 and a guanine for one allele and an adenine for the other allele at position 68974. This co-occurrence is a specific marker for the differential diagnosis of DLB versus AD. About 50% of DLB patients with a possible mis-diagnosis could be rescued by the use of this marker.

Another embodiment of the invention relates to a second genetic marker which is a genotype combination, AAAGCC8+K+. It is constituted by the specific genotypes of the polymorphic sites at positions 3687 (both alleles contain an adenine at this position), 4206 (one allele contains an adenine and the other a guanine), 4443 (both alleles contain a cytosine), 4780 to 4786 (at least one of the two alleles is constituted by 8 thymines) and 68974 (at least one of the two alleles contains an adenine). The determination of this genotype combination in demented patients serves as differential diagnostic marker providing the clinical diagnosis of DLB, but it may also serve as early diagnostic marker for DLB in asymptomatic individuals.

Another embodiment of the invention relates to a third genetic marker which is a genotype combination, AAAAC+77KW. It is constituted by the specific genotypes of the polymorphic sites at position 3687 (both alleles contain adenine at this position), 4206 (both alleles contain adenine at this position), 4443 (at least one of the two alleles contains a cytosine at this position), 4780 to 4786 (both alleles are constituted by 7 thymines) and 68974 (one allele contains an adenine and the other guanine). The determination of this genotype combination in demented patients serves as differential diagnostic marker providing the clinical diagnosis of DLB, but it may also serve as early diagnostic marker for DLB in asymptomatic individuals.

Advantageously, within the great heterogeneity of DLB and according to the examples, the method of the invention allows to differentially detect the 45-60% of DLB cases, which otherwise would be diagnosed as AD. This percentage of patients, difficult to diagnose in the clinical practice, will receive the correct diagnostic from the beginning of the disease. The specificity for the disease is of 99%. This represents the first specific marker for DLB.

Until now, the available tools in the state of the art did not allow the specific identification of DLB in the clinical practice. In this way, when the subject was diagnosed of AD, he was submitted to therapy with neuroleptics, which is the most adequate treatment for psychotic symptoms in AD but more than 50% of DLB patients exhibit an adverse reaction to this kind of treatment causing death in many cases. The method of the invention is of importance because it will enable the medical community to apply adequate treatment to patients suffering from DLB without the risks of an incorrect therapy. Therefore, applying the method of the invention, diagnostic specificity for DLB is increased as well as deaths caused by adverse effects of treatment with neuroleptics will be reduced.

Furthermore, as the method of the invention allows to specifically diagnosing patients with DLB, is it possible to have a defined group of patients to be included in a clinical trial.

By "diagnosis" in medicine it is meant the act or process of recognition of a disease or condition by its outward signs, symptoms, and underlying physiological/biochemical cause(s).

In the sense of this description, "alteration" in the BChE gene means any structural change in the nucleotide sequence considered as wild-type. Examples of alterations can include a single nucleotide polymorphism, a deletion, an insertion, a substitution or a duplication of one or more nucleotides, and a chemical modification on a nucleotide (e.g. methylation).

By "determining the genotype" in this description it is meant identifying the nucleotide in a given position.

In this description "a given nucleotide in one allele" means that the subject is heterozygote for that nucleotide in that gene, and "in both alleles", which is homozygote for that nucleotide.

According to the invention, the method includes determining the alterations indicated on BChE gene.

In accordance with the diagnostic method of the present invention, the analysis of DLB would be as follows: a patient with suspected onset of dementia and/or with a non-definitive clinical-familial evaluation would be diagnosed by a genetic test determining the alterations of the BChE gene described above. In the case of detecting the DLB specific genotypes, no additional tests or trial will be needed to diagnose correctly DLB. The direct application of genotyping represents an important save of money in the daily clinical practice.

The method of the invention is useful in the following suspected diagnosis: probable AD vs possible DLB; possible AD vs probable DLB; possible AD vs possible DLB; probable AD vs probable DLB; probable AD vs possible AD; possible DLB; and probable DLB. Physicians diagnose possible AD based on a full patient interview, covering personal and family medical history, combined with the outcome of any neurological, psychiatric, and lab tests conducted. Doctors are likely to expect AD when patient complains of a gradual progression of memory weakening, and when they are unable to find any other condition that could explain the memory loss. Doctors will be looking for disorders such as depression or hypothyroidism, neurological damage caused by stroke, or any medications that may be contributing to the loss of memory. An inability to uncover any contributory illness leads to the determination that AD is possible. Probable AD is a next step beyond possible Alzheimer's and means that a doctor is "relatively certain" that a patient has the disease.

Advantageously, the method of the invention allows a diagnosis of DLB without the need of obtaining samples by aggressive methods like a biopsy; and in this case a brain tissue microbiopsy. The method of the invention, being a genetic test, is performed on any biological sample removed from the subject, as blood, since it is applicable to any cell type of the body.

In another embodiment, the determination of the genotype is carried out by one of the techniques selected from the group consisting of primer-specific PCR multiplex followed by detection, multiplex allele specific primer extension, a microarray-based method, and dynamic allele-specific hybridization. In a particular embodiment, it is carried out by primer-specific PCR multiplex followed by detection.

The polymerase chain reaction (PCR) is the most widely used method for the *in vitro* amplification of nucleic acids. The PCR can be a real-time PCR, wherein the detection by labeled probes of the presence of the target genotypes is almost instantaneous to the amplification.

The amplification of the target polymorphisms can be performed by primer-specific PCR multiplex with following detection by polyacrylamide electrophoresis or by analysis with a genetic analyzer. Alternatively, various PCR reactions can be performed followed by agarose gel electrophoresis or by sequencing.

Determination of the genotype can be performed by Allele Specific Primer Extension (ASPE). This is a sequence specific enzymatic reaction technology that can be used to assay multiple SNPs in a single tube. The ASPE method involves two phases, an enzymatic reaction that determines the target genotype followed by a capture on solid microsphere surface for detection. Taking advantage of the solution phase kinetics, this technique allows sequence labeled microspheres to be used for detecting new templates. This is done with the help of an appropriate capture sequence attached to the allele specific oligonucleotide.

Optionally, detection may be carried out by DNA biochips/microarrays made with oligonucleotides deposited by any mechanism, by DNA biochips made, with oligonucleotides synthesized *in situ* by photolithography or any other mechanism. A microarray-based method that allow multiplex SNP genotyping in total human genomic DNA without the need for target amplification or complexity reduction can also be used for the genotyping of the BChE alterations. This direct SNP genotyping methodology requires no enzymes and relies on the high sensitivity of the gold nanoparticle probes. Specificity is derived from two sequential oligonucleotide hybridizations to the target by allele-specific surface-immobilized capture probes and gene-specific oligonucleotide-functionalized gold nanoparticle probes. The assay format is simple, rapid and robust pointing to its suitability for multiplex SNP profiling at the 'point of care'.

Furthermore, determination of the genotype can be performed by dynamic allele-specific hybridization (DASH), which represents the basis for throughput SNP genotyping in some laboratories. The core reaction principal of DASH is real-time (dynamic) tracking of allele-specific differences in the process of DNA denaturation. To achieve this, an oligonucleotide probe is first hybridized to the target DNA, a necessary component of essentially all genotyping methods. The target DNA comprises one strand of a PCR product immobilized onto a solid surface, and a single probe is used that is complementary to one of the target alleles. This assay concept was shown to be very precise (>99.9% accurate).

In a second aspect, the present invention provides a **set of primers which consists of the primers with sequences SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21,** for carrying out the method as defined above, which comprises determining the genotype of the alterations in BChE gene: **the polymorphic site at position 68974, the poly-thymine region at positions 4780 to 4786, and the polymorphic sites at position 3687, 4206, and 4443.**

In another aspect, the invention relates to the use of a set of primers as defined above, for the diagnosis of DLB.

The present invention also refers to the use of the polymorphic site at position 68974, in combination with one or more alterations in BChE gene selected from the group consisting of the poly-thymine region at positions 4780 to 4786, the polymorphic site at position 3687; the polymorphic site at position 4206; and the polymorphic site at position 4443, as marker for the diagnosis of dementia with Lewy bodies. In a particular embodiment, the polymorphic site at position 68974 is used in combination with the poly-thymine region at positions 4780 to 4786. In another embodiment, the polymorphic site at position 68974 is used in combination with the poly-thymine region at positions 4780 to 4786, and with the polymorphic sites at positions 3687, 4206, and 4443.

The invention also refers to a method of determining whether a subject will respond to treatment with neuroleptics, by analyzing the genotype of the above mentioned alterations in BChE gene. As the method allows determining whether a patient suffers from DLB or AD, is it possible to give the adequate treatment.

Throughout the description and claims the word "comprise" and variations of the word, such as "comprising", is not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein.

The following examples and drawings are provided by way of illustration, and are not intended to be limiting of the present invention.

### BRIEF DESCRIPTION OF THE DRAWINGS

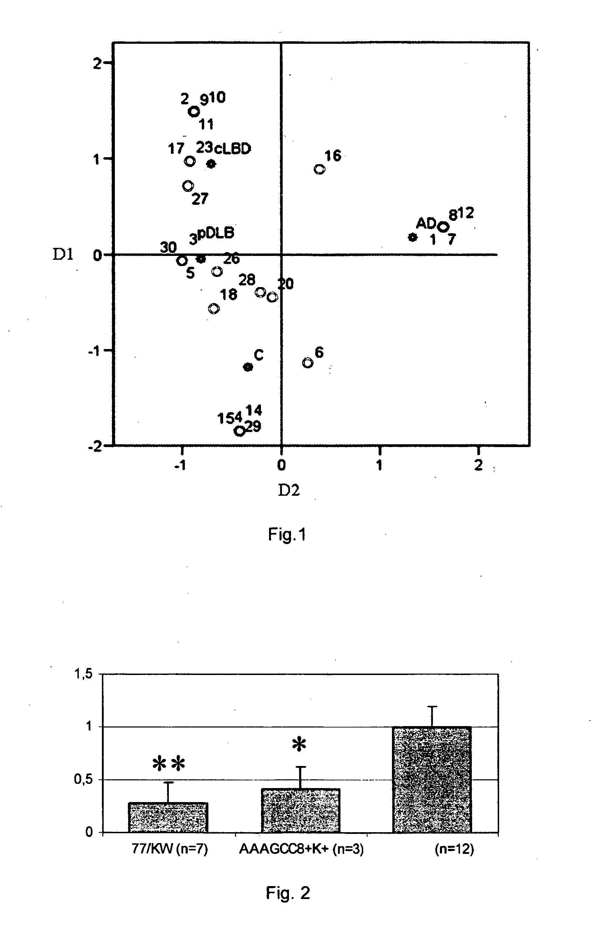

FIG. 1 shows the correspondence obtained for the four groups. Circles indicate the position of the different genotype combinations within the four-dimension-graph. Stars show the correspondent, genotype-combination-dependent localization of the disease and control groups. Their position clearly revealed significant differences between cDLB and controls, AD and controls, but also AD and cDLB. Whereas the control group was situated within the both dimension's negative quadrants, cLBD was situated within the first dimensions positive and second dimension negative quadrants and AD within the positive quadrant of the first and second dimensions. These results strongly indicate that the three groups presented significant differences respective to BChE genetics. That means that the disease-specific genotype combination may represent useful disease markers.
FIG. 2 shows the relative BChE expression levels in frontal cortex of three genotype-combination-dependent DLB groups: (1) genotype combination AAAGCC8+K+ carriers, n=4; (2) genotypes 77KW carriers, n=7; (3) rest of DLB patients, n=12. The results are represented as relative expression changes obtained by the deltadeltaCt method in comparison with the rest of DLB patients, where BChE expression was assumed as 1. Error bars represent the variance estimates. * Significant expression change between 1.5 and 2 times lower than the reference group, ** more than 2 times lower than the reference group. All DLB patients that carry one of the two DLB-specific BChE genotype combinations (AAAGCC8+K+ or AAAAC+77WW), present lower BChE expression levels than the rest of DLB patients. This diminished expression is similar to BChE expression levels in control brains, a fact indicating that DLB patients carrying AAAGCC8+K+ or AAAAC+77WW would not respond to usually used treatments with cholinesterase inhibitors. This finding moreover represents a specific characteristic for these DLB patients, defining a molecular subgroup of DLB.

### EXAMPLES

### Post-mortem samples

Post-mortem frontal cortex samples with their clinical and neuropathological diagnosis were facilitated by the University of Barcelona Neurological Tissue Bank and the Bellvitge Institute of Neuropathology Brain Bank (BrainNet Europe) according to the established rules of the local ethic committees. They corresponded to 24 brains with common Lewy body disease (cLBD) (age at death: 79.9, age range from 64 to 90; female : male ratio 1.5:1), to 12 brains with pure dementia with Lewy bodies (pDLB) (age at death: 74.4, age range from 60 to 80; female:male ratio 1:2), to 26 AD brains (age at death: 78.1, age range from 61 to 95; female:male ratio 1:1.1) and 23 control brains (age at death: 68.5, age range from 54 to 83; female:male ratio 1:1.1).

Neuropathologic examination revealed that all AD brains presented AD Braak and Braak stage VI. Braak and Braak is a staging to evaluate/quantify AD in brain. It is used by neuropathologists to evaluate density of amyloid plaques and neurofibrillary tangles. AD stages following Braak and Braak, I-VI: neurofibrillary tangles; A-C: amyloid plaques. Two of the cLBD samples corresponded to Braak and Braak stage III, three to Braak and Braak stage IV and the 19 remaining samples to stages V and VI. In pDLB brains Braak and Braak stages 0 to II were detected and in control samples AD related changes were absent. Whereas neither AD nor control brain showed PD-associated pathology, all pDLB as well as cLBD samples presented stages 5 and 6 corresponding to PD- related changes following classification of Braak and Braak.

### Clinically diagnosed samples

Blood samples were obtained from 223 AD patients (age: 71.1; age range from 49 to 86 years; female:male ratio 1:1.6) diagnosed in the Department of Neurology of our Hospital Germans Trias i Pujol, following NINCDS-ADRDA and DSM-IV criteria. Moreover, 59 age-matched control subjects (age: 68.8; age range from 46 to 91 years; female:male ratio 1:1.5).

In an additional experiment, a sample of 160 age-matched control subjects (age: 68.8; age range from 46 to 91 years; female:male ratio 1:1.5) was taken. The study was carried out after authorization of the Ethical Commitee from the Hospital and obtaining a signed informed consent.

### DNA extraction

DNA from frozen brain samples was extracted by the use of the TRI Reagent following manufacturer's instructions. TRI Reagent solution combines phenol and guanidine thiocyanate in a monophasic solution and it is used for the consecutive extraction of RNA, DNA and proteins from the same sample. After spectrophotometric determination of purity and concentration, DNA samples were stored at 4 °C until use. DNA extraction from blood was carried out by standard procedures based on DNA-binding on glass-filter membranes.

### BChE promoter sequencing

Since the BChE promoter sequence is constituted by approximately 5000 bp, three overlapping PCR fragments were amplified for their sequence analysis. PCR1 (primers BChEprom1UA and BChEprom1L; Table 1) yielded an 838 bp fragment spanning from position -1869 to position -1031. In PCR2 (primers BChEprom2UA and BChEpromS6; Table 1) a 837 bp fragment spanning from position -1152 to -315 and in PCR3 (primers BChEprom2UB and BChEprom2L; Table 1) a 688 bp fragment from position -473 to position +231 was obtained. PCR reactions with a final volume of 15 µl contained 1.7 mM MgCl₂, 200 µM of each dNTP (Ecogen), 2 pmol of each primer, 1 unit EcoTaq DNA polymerase (Ecogen) and approximately 300 ng of DNA. Standard PCR programs with annealing temperatures of 58 °C for PCR1 and 60 °C for PCRs 2 and 3 were constituted by 30 cycles for PCR1 and 35 cycles for PCRs 2 and 3.

| Table 1: Primers used for BChE promoter sequencing | | |
|---|---|---|
| primer name | primer sequence (5'→ 3') | SEQ ID NO |
| BChEprom1UA | TGATAGGCTGACCGTATGCT | SEQ ID NO: 2 |
| BChEprom1L | ACCTCATCAGATGAGAAAGC | SEQ ID NO: 3 |
| BChEprom2UA | TCTCTTGGAAGCAGTTGACAT | SEQ ID NO: 4 |
| BChEpromS6 | CCATTATAGCTTCAATCTGTGC | SEQ ID NO: 5 |
| BChEprom2UB | AGATACATATCAGAGACATCCATT | SEQ ID NO: 6 |
| BChEprom2L | GAAGAGATCACTCTCATCCC | SEQ ID NO: 7 |

PCR products were purifed by the use of the ExoSap-IT kit (GE Healthcare). Sequencing reactions were carried out with BigDye (BigDyeTM Terminator vs 1.1 Cycle Sequencing Kit, Perkin Elmer), 10 pmol/µl of the respective primer and 3.5 µl of the purified PCR product. After cycle sequencing and DNA precipitation, the sequences were obtained on the ABI PRISMTM3100 (Perkin Elmer).

### Analysis of BChE promoter polymorphisms

Four new polymorphisms were found in the promoter region of the BChE gene. Three of them, as well as the well known K-variant polymorphism, were studied using mutation-specific-PCR (MS-PCR): A3687G, A4206G, C4443T and BChE-K. Each PCR reaction with a final volume of 15 µl contained 1.7 mM MgCl₂, 200 µM of each dNTP (Ecogen), 2 pmol of each of the three primers (Table 2), 1 unit EcoTaq DNA polymerase (Ecogen) and 300 ng of DNA. Standard PCR programs of 35 cycles with annealing temperatures of 62 °C in the case of A3687G, BChE-K and of 57 °C in the case of A4206G, C4443T amplification were carried out. The obtained PCR fragments were separated on high resolution agarose gels. The A-allele of the BChE A3687G polymorphism was represented by a 153 bp and the G-allele by a 133 bp fragment. The K-allele was represented by a 149 bp fragment and the wild-type corresponding allele from the K-variant polymorphism, by a 169 bp band. A-allele of the BChE A4206G polymorphism was of 124 bp of length and the G-allele of 104 bp. Finally, in the case of the C4443T polymorphism, the T-allele corresponded to a 145 bp fragment and the C-allele to a 125 bp fragment.

The polyT polymorphism was constituted on different fragment sizes differing by only one nucleotide. Its genotyping was achieved by capillary electrophoresis on the ABI PRISM^{™} 3100 (Perkin Elmer) with a fluorochrome labeled primer (Table 2). The PCR was carried out under standard conditions using a 30-cycle program. The 7T-allele yielded a 196 bp and a 197 bp fragment represented the 8T-allele.

| Table 2: Primers used for BChE promoter genotyping | | | |
|---|---|---|---|
| Polymorp¹ | Primer name | primer sequence (5'→ 3') | SEQ ID NO |
| A3687G | BChE -1314U | TCTTGAACTCCCAGACTGAAGCA | SEQ ID NO: 8 |
| | BChE -1314G | TACACAAAAGGTACAGAATACAC | SEQ ID NO: 9 |
| | BChE -1314A | | SEQ ID NO: 10 |
| A4206G | BChE -795U | AAGTGCTCCACCTGCAAATATTA | SEQ ID NO: 11 |
| | BChE -795G | TAATCTTCTGTAAGTGATAGCC | SEQ ID NO: 12 |
| | BChE -795A | | SEQ ID NO: 13 |
| C4443T | BChE -558L | TGTCTCTGATATGTATCTCCTT | SEQ ID NO: 14 |
| | BChE -558CS | TCTTGACCAGAAAATTGTGGC | SEQ ID NO: 15 |
| | BCh E -558TL | | SEQ ID NO: 16 |
| BchE-K | BchE-4U | CTGTACTGTGTAGTTAGAGAAATTGGC | SEQ ID NO: 17 |
| | BchE-K | ATGGAATCCTGCTTTCCACTCCCATTCCGT | SEQ ID NO: 18 |
| | BchE-W | | SEQ ID NO: 19 |
| polyT | BChEpolyT-U | ²[FAM]TCAATAATAGCACTACTTTAGAATGA | SEQ ID NO: 20 |
| | BChEpolyT-L | AGGTAGTCTTCTAAGAATGAAGA | SEQ ID NO: 21 |

| | | | |
|---|---|---|---|
| Polymorp¹ : polymorphism name; ²[FAM] : 5'primer modification with the fluorochrome Fam. | | | |

### Statistical analyses

Correspondence analysis (CORRESPONDENCE, Version 1.1, Data Theory Scaling System Group (DTSS), Faculty of Social and Behavioral Sciences, Leiden University, The Netherlands) permitted obtaining the correspondence table in the case of the neuropathologically diagnosed patient group. The distribution of the genotype combinations for both patient groups (neuropathologically and clinically diagnosed) was calculated by the SSPS version 11.0.

### Match of clinical and neuropathology diagnosis

The match between both clinical and neuropathological diagnoses was first analyzed in the samples obtained from the Neurological Tissue Bank. Whereas 100% of AD patients coincided in their clinical and neuropathological diagnoses and 42% of pDLB patients received the diagnosis of DLB, only 17% of cLBD patients received the clinical diagnosis of DLB. Instead, 62% of them had been diagnosed as AD and 21% corresponded to other dementia related disorders. This observation fully correlates with the lack of diagnostic criteria for cLBD.

### The BChE K-variant

The BChE K-variant consist of a single nucleotide substitution from g to a at position 68974, where the g-allele is named W (wild type) and the a-allele K (mutated).

An interesting finding of this analysis was the overrepresentation of K-allele carrying genotypes in cLBD but also in pDLD and AD when compared to controls (0.62 in cLBD, 0.42 in pDLB and 0.38 in AD vs. 0.13 in controls, p<0.001, p=0.090 and p=0.058, respectively). The further genotypic analysis revealed that the KW genotype presented similar frequencies in AD and controls, was slightly elevated in pDLB, but about one third of cLBD samples were KW-genotype carriers (Table 3). Whereas neither the H- nor the J-variants were present in the studied samples, A-variant carrying genotypes were found at very low frequencies in the different diseases (0.04 cLBD, 0.08 in pDLB and 0.04 in AD vs 0 in controls; p=1, p=0,34 and p=1, respectively).

| Table 3: Allele and genotype distribution of the BChE K-variant polymorphism | | | | | |
|---|---|---|---|---|---|
| | Genotype frequencies | | | | |
| Disease | n¹ | WW | KW | KK | p² |
| cLBD | 24 | 0.38 | 0.29 | 0.33 | 0.003 |
| pDLB | 12 | 0.58 | 0.17 | 0.25 | 0.145 |
| AD | 26 | 0.62 | 0.08 | 0.30 | 0.047 |
| C | 23 | 0.87 | 0.09 | 0.04 | |

| | | | | | |
|---|---|---|---|---|---|
| ¹n: sample number; ²p: Exact test p value for genotypic comparisons between each disease and controls. | | | | | |

### Characterization of BChE promoter polymorphisms

BChE promoter sequencing revealed the presence of four polymorphisms previously not described. Three of them were single nucleotide changes: at position 3687, A was changed by G; A was substituted by G at position 4206 and C to T at position 4443. The fourth polymorphism corresponded to a polyT sequence of variable length and was located between positions 4780 and 4786. Of the two identified alleles, one was constituted by 7 Ts and the other by 8 Ts. Interestingly, the 8T-allele segregated with the K-allele of the common exon 4 polymorphism.

To ascertain if the polymorphisms showed a disease-specific association, allelic and genotypic frequencies for all four promoter polymorphisms were determined in neuropathologically diagnosed brain samples including cLBD, pDLB, AD and controls. First, the polymorphisms were analyzed independently and then, the existence of genotype combination was also tested.

The study of the A3687G polymorphism revealed an approximately three-fold increase of the AA genotype in AD when compared to cLBD, pDLB and controls (0.54 in AD vs. 0.21 in cLBD, p= 0.152; 0.16 in pDLB, p=0.298 and 0.13 in controls, p<0.001). In contrast G-allele carrying genotypes corresponding to the A4206G polymorphism were accumulated in cLBD, pDLB, as well as AD in comparison with controls (0.33 in cLBD, 0.17 in pDLB and 0.23 in AD vs. 0.04 in controls, p=0.023, p=0.262 and p=0.105, respectively). Although the accumulation of G-allele carrying genotypes was not disease specific, this accumulation seems to be of certain importance since G-allele carrying genotypes were almost absent in controls. The CC-genotype corresponding to the C4443T polymorphism was present at a very low frequency in pDLB when compared with cLBD as well as controls. Conversely, the frequency of the TC-genotype was elevated almost two fold in both pDLB and cLBD in comparison with AD and it was also significantly higher than in controls.

The frequencies of 8T-allele carrying genotypes showed about a two fold increase in cLBD, pDLB and AD in comparison with controls, but these differences were not significant (0.25 in cLBD, 0.17 in pDLB and 0.19 in AD vs. 0.09 in controls; p=0.245, p=0.591 and p=0.421, respectively).

### Analyses of genotype combinations in the post-mortem sample

### PolyT-77 K-variant KW genotype combination

Since the independent analysis of BChE genotypes did not reveal important differences between diseases, the analysis of all genotype combinations (GenComb) constituted by two polymorphisms revealed the co-occurrence of the KW/polyT-77 genotypes specifically in DLB. Whereas this genotype combination was absent in AD patients as well as controls, its frequency was of 0.3 in the cLBD group (6 KW/77 carriers of 24 patients) and of 0.17 in the pDLB group (2 KW/77 carriers of 12 patients), constituting a common frequency of 0.25 for DLB (Table 4).

| Table 4: Disease dependent distribution and frequencies of BChE-K variant and polyT genotype combinations | | | | | | |
|---|---|---|---|---|---|---|
| Group | n | WW/77 | KW/77 | KW/8+ | KK/77 | KK/8+ |
| cLBD | 24 | 0.45 | 0.3 | 0.0 | 0.15 | 0.1 |
| pDLB | 12 | 0.58 | 0.17 | 0.0 | 0.08 | 0.17 |
| AD | 26 | 0.47 | 0.0 | 0.1 | 0.26 | 0.16 |
| C | 23 | 0.87 | 0.0 | 0.09 | 0.04 | 0.0 |

### Correspondence analysis

The further analysis of the distribution, frequency and specificity of the GenComb constituted by the four promoter and the K-variant polymorphisms were studied. The correspondence analysis of these GenComb revealed BChE-based genetic differences between cLBD, AD and controls as well as between cLBD and AD. These differences could be clearly represented in a four-quadrant diagram (FIG. 1). Whereas the control group was situated within the first and second dimension's negative quadrant, cLBD was situated within the first dimension negative and the second dimensions positive quadrant and AD within the positive quadrant of both dimensions. These results strongly indicated that the three groups were unrelated respective to BChE genetics, and correspondently the existence of disease-specific GenComb.

### Common genotype combinations

The first, overall analysis revealed the presence of 31 different GenComb (Table 5). Since most of those (64.5%) were present in one or two samples only, their frequency was very low (0.01 and 0.02). The most frequent GenComb (N° 20), constituted by the homozygous wild-type genotypes of all polymorphisms, represented 17.6% of the whole sample and was present at similar frequencies in all groups. The other two most frequent GenComb (N°18 and 20) were also unspecific because of their presence in all four groups. In contrast to AD and controls, both types of LB dementia were the most heterogeneous diseases respective to BChE genetics (Table 5). Interestingly, AD was characterized by more disease-specific GenComb than both DLBs and controls (Table 5).

| Table 5: Correspondence table obtained for the neuropathological sample | | | | | | |
|---|---|---|---|---|---|---|
| Haplotype | | | | | | |
| | Genotype combination | C | AD | cLBD | pDLB | active margin |
| 1 | AAAACC77KK | 0 | 2 | 0 | 0 | 2 |
| 2 | AAAACC77KW | 0 | 0 | 1 | 0 | 1 |
| 3 | AAAACC77WW | 0 | 0 | 0 | 1 | 1 |
| 4 | AAAACC78KW | 1 | 0 | 0 | 0 | 1 |
| 5 | AAAATC77KK | 0 | 0 | 0 | 1 | 1 |
| 6 | AAAATC77WW | 2 | 1 | 0 | 0 | 3 |
| 7 | AAAATT77KK | 0 | 1 | 0 | 0 | 1 |
| 8 | AAAATT77WW | 0 | 7 | 0 | 0 | 7 |
| 9 | AAAGCC78KK | 0 | 0 | 2 | 0 | 2 |
| 10 | AAAGCC78KW | 0 | 0 | 1 | 0 | 1 |
| 11 | AAAGCC88KK | 0 | 0 | 1 | 0 | 1 |
| 12 | AAAGTC78KK | 0 | 2 | 0 | 0 | 2 |
| 13 | AAAGTT78KK | 0 | 1 | 0 | 0 | 1 |
| 14 | AGAACC77KK | 1 | 0 | 0 | 0 | 1 |
| 15 | AGAACC77WW | 1 | 0 | 0 | 0 | 1 |
| 16 | AGAATC77KK | 0 | 2 | 2 | 0 | 4 |
| 17 | AGAATC77KW | 0 | 0 | 2 | 1 | 3 |
| 18 | AGAATC77WW | 4 | 0 | 2 | 2 | 8 |
| 19 | AGAATT77KW | 0 | 0 | 1 | 0 | 1 |
| 20 | AGAATT77WW | 6 | 3 | 3 | 1 | 13 |
| 21 | AGAGTC77KK | 0 | 0 | 1 | 0 | 1 |
| 22 | AGAGTC77KW | 0 | 0 | 1 | 0 | 1 |
| 23 | AGAGTC78KK | 0 | 0 | 2 | 1 | 3 |
| 24 | AGAGTC78KW | 0 | 2 | 0 | 0 | 2 |
| 25 | GGAACC77WW | 0 | 1 | 0 | 0 | 1 |
| 26 | GGAATC77WW | 1 | 0 | 1 | 0 | 2 |
| 27 | GGAATT77KW | 0 | 0 | 1 | 1 | 2 |
| 28 | GGAATT77WW | 6 | 3 | 3 | 3 | 15 |
| 29 | GGAGCC78KW | 1 | 0 | 0 | 0 | 1 |
| 30 | GGAGTC78KK | 0 | 0 | 0 | 1 | 1 |
| 31 | GGAGTT77WW | 0 | 1 | 0 | 0 | 1 |
| | active margin | 23 | 26 | 24 | 12 | 85 |

### Disease-specific genotype combinations

When analyzed by diseases, two important disease-specific GenComb could be detected. On one hand, the GenComb AAAATT77WW was only present in AD samples and at the relative high frequency of 0.29. The related GenComb AAAATT77KK was also found in an AD sample, underlining disease-specificity (Table 6). The fact that polyT-8T allele co-segregates with the K-allele permitted to establish GenComb 9-11 as related and to define them as the common GenComb AAAGCC8+K+. This GenComb was the most frequent (16.7%) disease-specific GenComb found in cLBD (Table 6). The three unrelated pDLB specific GenComb (N° 3, 5 and 30) had the low frequency of 0.08 (present each of them in one patient only). Four GenComb were found specifically in controls. Although two of them (N° 14 and 15) were related, their frequency did not overcome 0.1.

| Table 6: Disease dependent distribution and frequencies of BChE-K variant and polyT genotype combinations in neupathologically diagnosed patients | | | |
|---|---|---|---|
| Group | n | AAAATT77WW/KK | AAAGCC8+K+ |
| cLBD | 24 | 0 | 0.17 |
| pDLB | 12 | 0 | 0 |
| AD | 26 | 0.3 | 0 |
| C | 23 | 0 | 0 |

### Analyses of genotype combinations in the sample with clinical diagnosis

To confirm the data obtained by the study of neuropathologically diagnosed post-mortem AD, DLB and control samples, we also studied a group of 230 AD patients as well as 59 controls. The AD patients had been diagnosed between 1998 and 2002. The latest guidelines for clinical DLB diagnosis had been established in 2005, so it can be expected that between 10 and 25% of these AD patients should be misdiagnosed DLB patients.

### PolyT-77 K-variant KW genotype combination

The analysis of the co-occurrence of the variant-K and polyT genotypes revealed similar frequencies of the KW/polyT-77 genotype co-occurrence in both, the AD (0.19) and the control groups (0.15) (Table 7).

| Table 7: Disease dependent distribution and frequencies of BChE-K variant and polyT genotype combinations | | | | | | |
|---|---|---|---|---|---|---|
| Group | N | WW/77 | KW/77 | KW/8+ | KK/77 | KK/8+ |
| AD | 223 | 0.63 | 0.19 | 0.11 | 0.004 | 0.004 |
| C | 60 | 0.70 | 0.15 | 0.17 | 0 | 0 |

Nevertheless, the clinical histories of the 42 patients carrying the KW/77 genotypes were revised and all 42 patients presented symptoms compatible with DLB diagnosis.

Although the genotype co-occurrence KW/77 presents similar frequencies in patients and controls, it is characterized by an elevated specificity to genetically distinguish AD and DLB. When also taken into account that 20-40% of the AD patient group are misdiagnosed DLB patients, the disease-specific frequency of KW/77 genotypes would increase up to more than 50%.

### Identification of a second DLB-specific BChE-genotype combination

Due to the results of BChE expression analyses, a reanalysis was performed for all genotype combinations in the clinical sample. Carriers of combined genotypes 77KW were found with similar frequencies in both the AD and control groups. Instead, GenComb AAAAC+77KW, was present in 11 patients of the AD group (frequency of 0.05) and in 2 individuals of the control group (frequency of 0.012). The revision of the clinical histories also revealed in all of them symptoms compatible with DLB. Similar to GenComb AAAGCC8+K+, AAAAC+77KW-frequency would range between 12 - 25%, taken into account that 20-40% of our AD group, are actually DLB patients. The specificity of AAAAC+77KW would be of 98.7% and its sensitivity up to 30%.

If then combining both GenComb, the testing of BChE genotype combinations would allow the detection of DLB cases with up to 60% of sensitivity being the specificity of 96.8%.

### Correspondence analysis

Since the sample with clinical diagnosis was represented by two groups only, the AD and the control group, results of the correspondence analysis were obtained in contingency tables (Table 8 and Table 10). Taking into account that five polymorphisms were included within the genotype combination to be analyzed, it was very surprisingly to find only 25 different genotype combinations (from 243 possible genotype combinations) in that sample constituted by 289 individuals (Table 8). 64% of all detected genotype combinations had been also observed in the neuropathologically diagnosed sample.

| Table 8: Correspondence table obtained for the clinical sample (59 control individuals) | | | | |
|---|---|---|---|---|
| | Haplotype | | | |
| | Genotype combination | AD | C | active margin |
| 1 | AAAACC77KW | 7 | 1 | 8 |
| 2 | AAAACC77WW | 2 | 0 | 2 |
| 3 | AAAACC78KK | 1 | 0 | 1 |
| 4 | AAAATC77KW | 4 | 0 | 4 |
| 5 | AAAATC77WW | 5 | 1 | 6 |
| 6 | AAAATT77KK | 1 | 0 | 1 |
| 7 | AAAATT77WW | 5 | 2 | 7 |
| 8 | AAAGCC77WW | 0 | 1 | 1 |
| 9 | AAAGCC78KK | 5 | 0 | 5 |
| 10 | AAAGCC78KW | 4 | 0 | 4 |
| 11 | AAAGCC88KK | 4 | 0 | 4 |
| 12 | AAAGCC88KW | 1 | 0 | 1 |
| 13 | AAAGTC78KW | 4 | 5 | 9 |
| 14 | AGAACC77KW | 2 | 0 | 2 |
| 15 | AGAATC77KW | 24 | 5 | 29 |
| 16 | AGAATC77WW | 24 | 4 | 28 |
| 17 | AGAATC78KW | 2 | 0 | 2 |
| 18 | AGAATT77KW | 4 | 0 | 4 |
| 19 | AGAATT77WW | 32 | 13 | 45 |
| 20 | AGAGTC77KW | 0 | 1 | 1 |
| 21 | AGAGTC78KW | 19 | 4 | 23 |
| 22 | AGAGTT77WW | 1 | 0 | 1 |
| 23 | AGAGTT78KW | 1 | 0 | 1 |
| 24 | GGAATT77KW | 2 | 1 | 3 |
| 25 | GGAATT77WW | 76 | 21 | 97 |
| | active margin | 230 | 59 | 289 |

### Common genotype combinations of Table 8

The five most frequent genotype combinations were: combination 25 with a frequency of 0.34 (0.34 in AD and 0.35 in controls), combination 19 with a frequency of 0.16 (0.14 in AD and 0.22 in controls), 15 with a frequency of 0.10 (0.11 in AD and 0.08 in controls), combination 16 with a frequency of 0.10 (0.11 in AD and 0.07 in controls) and, finally, combination 21 with a frequency of 0.08 (0.08 in AD and 0.07 in controls; Table 8). The most frequent genotype combination was the same in both: the neuropathological and the clinical samples. Moreover, other two genotype combinations coincided as most frequent in both samples (Tables 5 and 8).

### Disease-specific genotype combinations of Table 8

Analyzing the distribution of the GenComb earlier detected as DLB- and AD-specific, we found a much lower representation for both. On one hand, the GenComb AAAATT77WW was only present in 2% of AD samples but, on the other hand it was also detected in 3.3% of the control group. The related GenComb AAAATT77KK was only found in one AD sample (0.4%) (Table 9).

GenComb 9-11 coincided with GenComb of the neuropathological sample and were also defined as the common GenComb AAAGCC8+K+. This GenComb was present with a frequency of 0.06 (6%) only in the AD group (Table 9).

| Table 9: Disease dependent distribution and frequencies of BChE-K variant and polyT genotype combinations in clinically diagnosed patients | | | |
|---|---|---|---|
| Group | n | AAAATT77WW/KK | AAAGCC8+K+ |
| AD | 223 | 0.03 | 0.06 |
| C | 60 | 0.03 | 0 |

Taking into account that the AD patients had been clinically diagnosed about 8 years ago, much before the establishment of the new guidelines for DLB diagnosis, the clinical histories of the 13 patients carrying the GenComb AAAGCC8+K+ were revised. All 13 patients presented symptoms compatible with possible or probable DLB, corroborating AAAGCC8+K+ as specific DLB marker.

When furthermore taking into account that 20-40% of the AD patient group are misdiagnosed DLB patients, the disease-specific frequency of AAAGCC8+K+ increases and would range between 15-28%.

| Table 10: Correspondence table obtained for the clinical sample (160 control individuals) | | | |
|---|---|---|---|
| **Genotype combination** | **C** | **AD** | **Active margin** |
| 1 AAAACC77KW | 2 | 7 | 9 |
| 2 AAAACC77WW | 3 | 2 | 5 |
| 3 AAAACC78KK | 0 | 1 | 1 |
| 4 AAAATC77KW | 0 | 4 | 4 |
| 5 AAAATC77WW | 1 | 5 | 6 |
| 6 AAAATT77KK | 0 | 1 | 1 |
| 7 AAAATT77WW | 5 | 5 | 10 |
| 8 AAAGCC77WW | 1 | 0 | 1 |
| 9 AAAGCC78KK | 1 | 5 | 6 |
| 10 AAAGCC78KW | 1 | 4 | 5 |
| 11 AAAGCC88KK | 1 | 1 | 2 |
| 12 AAAGCC88KW | 0 | 1 | 1 |
| 13 AAAGTC78KW | 6 | 4 | 10 |
| 14 AGAACC77KK | 1 | 0 | 1 |
| 15 AGAACC77KW | 2 | 2 | 4 |
| 16 AGAACC77WW | 1 | 0 | 1 |
| 17 AGAATC77KK | 1 | 0 | 1 |
| 18 AGAATC77KW | 23 | 24 | 47 |
| 19 AGAATC77WW | 20 | 24 | 44 |
| 20 AGAATC78KW | 3 | 0 | 3 |
| 21 AGAATT77KW | 1 | 2 | 3 |
| 22 AGAATT77WW | 25 | 32 | 57 |
| 23 AGAGTC77KW | 1 | 0 | 1 |
| 24 AGAGTC77WW | 1 | 0 | 1 |
| 25 AGAGTC78KW | 7 | 19 | 26 |
| 26 AGAGTT77WW | 0 | 1 | 1 |
| 27 AGAGTT78KW | 0 | 1 | 1 |
| 28 GGAATT77KW | 4 | 2 | 6 |
| 29 GGAATT77WW | 49 | 76 | 125 |
| **Active margin** | 160 | 223 | 383 |

### Common genotype combinations of Table 10

The five most frequent genotype combinations were: combination 29 with a frequency of 0.33 (0.34 in AD and 0.31 in controls), combination 22 with a frequency of 0.15 (0.14 in AD and 0.16 in controls), 18 with a frequency of 0.12 (0.11 in AD and 0.14 in controls), combination 19 with a frequency of 0.11 (0.11 in AD and 0.12 in controls) and, finally, combination 25 with a frequency of 0.07 (0.08 in AD and 0.05 in controls; Table 10). The most frequent genotype combination was the same in both: the neuropathological and the clinical samples. Moreover, other two genotype combinations coincided as most frequent in all samples (Tables 5, 8 and 10).

### Disease-specific genotype combinations

Analyzing the distribution of the GenComb earlier detected as DLB- and AD-specific, we found a much lower representation for both. On one hand, the GenComb AAAATT77WW was only present in 2% of AD samples but, on the other hand it was also detected in 3.1% of the control group. The related GenComb AAAATT77KK was only found in one AD sample (0.4%) (Table 11).

GenComb 9-12 coincided with GenComb of the neuropathological sample and were also defined as the common GenComb AAAGCC8+K+. This GenComb was present with a frequency of 0.06 (6%) in the AD group and of 0.01 in controls (Table 11).

| Table11: Disease dependent distribution and frequencies of BChE-K variant and polyT genotype combinations in clinically diagnosed patients | | | |
|---|---|---|---|
| Group | N | AAAATT77WW/KK | AAAGCC8+K+ |
| AD | 223 | 0.02 | 0.06 |
| C | 60 | 0.03 | 0.01 |

Taking into account that the AD patients had been clinically diagnosed about 8 years ago, much before the establishment of the new guidelines for DLB diagnosis, the clinical histories of the 13 patients carrying the GenComb AAAGCC8+K+ were revised. All 13 patients presented symptoms compatible with possible or probable DLB, corroborating AAAGCC8+K+ as specific DLB marker.

When furthermore taking into account that 20-40% of the AD patient group are misdiagnosed DLB patients, the disease-specific frequency of AAAGCC8+K+ increases and would range between 15-28%.

### BChE expression in DLB in dependency on genotype combinations

### RNA isolation and reverse transcription

TRI -Reagent (MRC, Cincinnati, USA) was used for RNA isolation according to the manufacturer's protocol. Briefly, 100 mg tissue samples were homogenized in a 1.5 ml tube with a sterile piston in 1.0 ml of TRI-Reagent. Homogenates were incubated 5 min at room temperature and then centrifuged at 12,000 g for 10 min at 4 °C to pellet insoluble material and high-molecular-weight DNA. After phase separation, RNA was precipitated with isopropanol and resuspended in an appropriate volume of DEPC-treated water. RNA quantity was determined spectrophotometrically at A260, RNA purity was ascertained from optical density ratio at 260 nm and 280 nm. RNA integrity was ascertained by the use of the Agilent 2100 Bioanalyzer (Agilent Technologies, Santa Clara, USA). Only samples with RIN values higher than 6 were stored at -80 °C until use.

First-strand cDNA synthesis was carried out using Ready-to-go^{™} You-Prime First-Strand Beads (Amersham Pharmacia Biotech, Uppsala, Sweden). Two mg of RNA were incubated with random hexamers and the First-Strand Beads at 37 °C during 1 hour. The resulting cDNA was either immediately used for PCR or stored at -20 °C until use.

### Real time PCR

The relative expression of BChE mRNA was determined using a Rotor-Gene 6000 (Corbett Life Science, Sydney, Australia). A QuantiTect SYBR Green PCR Kit (QiaGen, Hilden, Germany) was used to minimize the primer-dimer content. Fifteen ml reactions further contained 16 pmol of each primer (BChE 2U GAGTAGATCCATAGTGAAACGG, SEQ ID NO: 22, and BChE 6LRNA CAGCGATGGAATCCTGCTTT, SEQ ID NO: 23) and 1 ml of cDNA. To study relative BChE amounts, two housekeeping genes were also analyzed, beta-actin (primers: beta-actin U2 TCTACAATGAGCTGCGTGTG, SEQ ID NO: 24, and beta-actin L3 TAGATGGGCACAGTGTGGGT, SEQ ID NO: 25) and beta-glucuronidase (GUS; primers: GUS-U1 ATGTGGTTGGAGAGCTCATT, SEQ ID NO: 26 and GUS-L2 TGTCTCTGCCGAGTGAAGAT, SEQ ID NO: 27) (M. Barrachina et al., "TaqMan PCR assay in the control of RNA normalization in human post-mortem brain tissue", Neurochem Int 2006, vol. 49, pp. 276-84).

After a 15-minutes-denaturation step, followed by 30 seconds of annealing at 56 °C for all BChE, GUS and beta-actin, end fluorescence data were acquired during a standard 72 °C extension. A final melting analysis was run for all products to determine the specific amplification. Relative expression data were achieved by the deltadelta Ct method based on the assumption of similar PCR efficiencies to analyze relative gene expression (T.D. Schmittgen et al., "Analyzing real-time PCR data by the comparative C(T) method", Nat Protoc 2008, vol. 3, pp 1101-8). Therefore, different primer pairs of each gene and isoform were tested to obtain fragments with a length between 100 and 150 base pairs that become amplified with similar efficiencies. Since PCR efficiencies can vary in each run, a standard curve was included in each and not only in the initial run. Only runs with similar efficiencies together with a correct standard curve (R>0.99 and R^2>0.99) were suitable for further analyses. Standard curves were generated by amplifying the same serially diluted cDNA control sample. All assays were performed twice and independently to assure their reproducibility and minimize possible errors, including additionally a negative control in each run.

### Results

The analysis of relative expression levels of BChE in brain in dependency on the presence of the genotype combinations showed that carriers of the genotype combination 77/KW showed a decrease in the BChE expression in brain of more than three times; and carriers of the genotype combination AAAGCC8+K+ showed a decrease in the expression higher than 50%. This defines a subgroup in DLB which will probably not respond to treatment with cholinesterase and inhibitors, and specifically butyrylcholinesterase inhibitors, since the BChE is already decreased.

### REFERENCES CITED IN THE DESCRIPTION

- I.G. McKeith, "Consensus guidelines for the clinical and pathologic diagnosis of dementia with Lewy bodies (DLB): report of the Consortium on DLB International Workshop", J. Alzheimer's Dis. 2006, vol. 9, pp. 417-23
- F. Parmo-Folloni et al., "Two new mutations of the human BCHE gene (IVS3-14T>C and L574fsX576)" Chemico-Biological Interactions 2008, vol. 175, pp. 135-7
- A.B. Singleton et al., "Butyrylcholinesterase K: an association with dementia with Lewy bodies", Lancet 1998, vol. 351, pp. 1818.
- R. Lane et al., "BuChE-K and APOE epsilon4 allele frequencies in Lewy body dementias, and influence of genotype and hyperhomocysteinemia on cognitive decline", Mov. Disord. 2009, vol. 24, pp. 392-400.
- W. Maetzler et al., "No differences of butyrylcholinesterase protein activity and allele frequency in Lewy body diseases" Neurobiol. Dis. 2009, vol. 35, pp. 296-301
- M. Barrachina et al., "TaqMan PCR assay in the control of RNA normalization in human post-mortem brain tissue", Neurochem Int 2006, vol. 49, pp. 276-84
- T.D. Schmittgen et al., "Analyzing real-time PCR data by the comparative C(T) method", Nat Protoc 2008, vol. 3, pp 1101-8

## Claims

1. An in vitro method for the diagnosis of dementia with Lewy bodies comprising determining in a biological sample from a subject, the genotype of the following alterations in butyrylcholinesterase (BChE) gene:
the polymorphic site at position 68974 in NCBI Accession Number NG_009031 (i.e. position 934 in SEQ ID NO: 28); and
the poly-thymine region at positions 4780 to 4786 in NCBI Accession Number NG_009031 (i.e. positions 4780-4786 in SEQ ID NO: 1).

2. The method according to claim 1, which further comprises determining the genotype of the following alterations in BChE gene:
the polymorphic site at position 3687,
the polymorphic site at position 4206, and
the polymorphic site at position 4443, said positions with reference to NCBI Accession Number NG_009031 (i.e. positions 3687, 4206 and 4443 respectively in SEQ ID NO: 1).

3. The method according to claim 1, wherein the genotype is:
seven thymines at positions 4780 to 4786 for both alleles; and
a guanine for one allele and an adenine for the other allele at position 68974;
being this genotype indicative of dementia with Lewy bodies and
distinguishing from Alzheimer disease.

4. The method according to claim 2, wherein the genotype is:
an adenine for one allele at position 68974;
eight thymines for one allele at positions 4780 to 4786;
adenine for both alleles at position 3687;
an adenine for one allele and a guanine for the other allele at position 4206;
and
cytosine for both alleles at position 4443;
being this genotype indicative of dementia with Lewy bodies and
distinguishing from Alzheimer disease.

5. The method according to claim 2, wherein the genotype is: an adenine for one allele and a guanine for the other allele at position 68974;
seven thymines for both alleles at positions 4780 to 4786;
an adenine for both alleles at position 3687;
an adenine for both alleles at position 4206; and
cytosine for one allele at position 4443;
being this genotype indicative of dementia with Lewy bodies and distinguishing from Alzheimer disease.

6. The method according to any of the claims 1-5, wherein the determination of the genotype is carried out by one of the techniques selected from the group consisting of primer-specific PCR multiplex followed by detection, multiplex allele specific primer extension, a microarray-based method, and dynamic allele-specific hybridization.

7. The method according to claim 6, wherein the determination is carried out by amplification by primer-specific PCR multiplex followed by detection.

8. The method according to any of the claims 1-7, wherein the biological sample is a blood sample.

9. A set of primers for carrying out the method as defined in any of claims 2, 4 or 5, which consists of the primers with sequences: SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21.

10. Use of the set of primers as defined in claim 9, for the diagnosis of dementia with Lewy bodies.

11. Use of a method for genotyping the polymorphic site at position 68974 in NCBI Accession Number NG_009031 (i.e. position 934 in SEQ ID NO: 28), in combination with one or more alterations in BChE gene selected from the group consisting of the polythymine region at positions 4780 to 4786, the polymorphic site at position 3687; the polymorphic site at position 4206; and the polymorphic site at position 4443, to diagnose dementia with Lewy bodies, said positions with reference to NCBI Accession Number NG_009031 (i.e. positions 3687, 4206, 4443 and 4780-4786 respectively in SEQ ID NO: 1).

12. The use according to claim 11, wherein the polymorphic site at position 68974 is genotyped in combination with the poly-thymine region at positions 4780 to 4786.

13. The use according to claim 11, wherein the polymorphic site at position 68974 is genotyped in combination with the poly-thymine region at positions 4780 to 4786, the polymorphic site at position 3687; the polymorphic site at position 4206; and the polymorphic site at position 4443.

## Patentansprüche

1. Ein *in-vitro-Verfahren* zur Diagnose von Lewy-Body-Demenz, umfassend das Feststellen des Genotyps von den folgenden Veränderungen im Butyrylcholinesterase- (BChE)-Gen in einer biologischen Probe von einem Subjekt:
die polymorphe Stelle an Position 68974 in der NCBI Accession Number NG_009031 (d. h. die Position 934 in SEQ ID NO: 28); und
die Poly-Thymin-Region an den Positionen 4780 bis 4786 in der NCBI Accession Number NG_009031 (d. h. die Positionen 4780-4786 in SEQ ID NO: 1).

2. Verfahren gemäß Anspruch 1, welches weiterhin das Feststellen des Genotyps von den folgenden Änderungen im BChE-Gen umfasst:
die polymorphe Stelle an Position 3687,
die polymorphe Stelle an Position 4206, und
die polymorphe Stelle an Position 4443, wobei die Positionen auf die NCBI Accession Number NG_009031 verweisen (d. h. die Positionen 3687, 4206 beziehungsweise 4443 in SEQ ID NO: 1).

3. Verfahren gemäß Anspruch 1, worin der Genotyp ist:
sieben Thymine an den Positionen 4780 bis 4786 für beide Allele; und
ein Guanin für ein Allel und ein Adenin für das andere Allel an Position 68974;
wobei dieser Genotyp für Lewy-Body-Demenz spricht und von der Alzheimer-Krankheit unterscheidet.

4. Verfahren gemäß Anspruch 2, worin der Genotyp ist:
ein Adenin für ein Allel an der Position 68974;
acht Thymine für ein Allel an den Positionen 4780 bis 4786;
Adenin für beide Allele an Position 3687;
ein Adenin für ein Allel und ein Guanin für das andere Allel an Position 4206;und
Cytosin für beide Allele an Position 4443;
wobei dieser Genotyp für Lewy-Body-Demenz spricht und von der Alzheimer-Krankheit unterscheidet.

5. Verfahren gemäß Anspruch 2, worin der Genotyp ist: ein Adenin für ein Allel und ein Guanin für das andere Allel an der Position 68974;
sieben Thymine für beide Allele an den Positionen 4780 bis 4786;
ein Adenin für beide Allele an Position 3687;
ein Adenin für beide Allele an Position 4206; und
Cytosin für ein Allel an Position 4443;
wobei dieser Genotyp für Lewy-Body-Demenz spricht und von der Alzheimer-Krankheit unterscheidet.

6. Verfahren gemäß einem der Ansprüche 1-5, worin die Feststellung des Genotyps durch eine der Techniken ausgeführt wird, ausgewählt aus der Gruppe, bestehend aus primerspezifischer Multiplex-PCR, gefolgt vom Nachweis, multiplexer allelspezifischer Primer Extension, einem mikroarraybasierten Verfahren und dynamischer allelspezifischer Hybridisierung.

7. Verfahren gemäß Anspruch 6, worin die Feststellung durch Amplifiktion mittels primerspezifischer Multiplex-PCR, gefolgt vom Nachweis, ausgeführt wird.

8. Verfahren gemäß einem der Ansprüche 1-7, worin die biologische Probe eine Blutprobe ist.

9. Primernsatz zum Ausführen des Verfahrens wie in einem der Ansprüche 2, 4 oder 5 definiert, welches aus den Primern mit den Sequenzen besteht: SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 und SEQ ID NO: 21.

10. Verwendung des Primernsatz wie in Anspruch 9 definiert für die Diagnose von Lewy-Body-Demenz.

11. Verwendung eines Verfahrens zum Genotypisieren der polymorphen Stelle an Position 68974 in NCBI Accession Number NG_009031 (d. h. der Position 934 in SEQ ID NO: 28), in Kombination mit einer oder mehreren Veränderungen im BChE-Gen, ausgewählt aus der Gruppe, bestehend aus der Poly-Thymin-Region an den Positionen 4780 bis 4786, der polymorphen Stelle an Position 3687; der polymorphen Stelle an Position 4206; und der polymorphen Stelle an Position 4443, um Lewy-Body-Demenz zu diagnostizieren, wobei die Positionen auf NCBI Accession Number NG_009031 verweisen (d. h. die Positionen 3687, 4206, 4443 beziehungsweise 4780-4786 in SEQ ID NO: 1).

12. Verwendung gemäß Anspruch 11, worin die polymorphe Stelle an Position 68974 in Kombination mit der Poly-Thymin-Region an den Positionen 4780 bis 4786 genotypisiert wird.

13. Verwendung gemäß Anspruch 11, worin die polymorphe Stelle an Position 68974 in Kombination mit der Poly-Thymin-Region an den Positionen 4780 bis 4786, der polymorphen Stelle an Position 3687; der polymorphen Stelle an Position 4206 und der polymorphen Stelle an Position 4443 genotypisiert wird.

## Revendications

1. Procédé in vitro de diagnostic de la démence à corps de Lewy comprenant la détermination, dans un échantillon biologique d'un sujet, du génotype des modifications suivantes du gène de la butyrylcholinestérase (BChE) :
le site polymorphe à la position 68974 dans le numéro d'accès NCBI NG_009031 (en d'autres termes, position 934 dans SEQ ID NO : 28) ; et
la région poly-thymine aux positions 4780 à 4786 dans le numéro d'accès NCBI NG_009031 (en d'autres termes, positions 4780 à 4786 dans SEQ ID NO : 1).

2. Procédé selon la revendication 1, qui comprend en outre la détermination du génotype des modifications suivantes dans le gène BChE :
le site polymorphe à la position 3687,
le site polymorphe à la position 4206, et
le site polymorphe à la position 4443, lesdites positions faisant référence au numéro d'accès NCBI NG_009031 (en d'autres termes, positions 3687, 4206 et 4443 respectivement dans SEQ ID NO : 1).

3. Procédé selon la revendication 1, dans lequel le génotype est :
sept thymines aux positions 4780 à 4786 pour les deux allèles ; et
une guanine pour un allèle et une adénine pour l'autre allèle à la position 68974 ;
ce génotype étant indicateur de démence à corps de Lewy et permettant la différenciation par rapport à la maladie d'Alzheimer.

4. Procédé selon la revendication 2, dans lequel le génotype est :
une adénine pour un allèle à la position 68974 ;
huit thymines pour un allèle aux positions 4780 à 4786 ;
une adénine pour les deux allèles à la position 3687 ;
une adénine pour un allèle et une guanine pour l'autre allèle à la position 4206 ; et
une cytosine pour les deux allèles à la position 4443 ;
ce génotype étant indicateur de démence à corps de Lewy et permettant la différenciation par rapport à la maladie d'Alzheimer.

5. Procédé selon la revendication 2, dans lequel le génotype est :
une adénine pour un allèle et une guanine pour l'autre allèle à la position 68974 ;
sept thymines pour les deux allèles aux positions 4780 à 4786 ;
une adénine pour les deux allèles à la position 3687 ;
une adénine pour les deux allèles à la position 4206 ; et
une cytosine pour un allèle à la position 4443 ;
ce génotype étant indicateur de démence à corps de Lewy et permettant la différenciation par rapport à la maladie d'Alzheimer.

6. Procédé selon l'une quelconque des revendications 1 à 5, dans lequel la détermination du génotype est réalisée par l'une des techniques sélectionnées dans le groupe constitué d'une PCR multiplexe utilisant des amorces spécifiques suivie de la détection, de l'extension d'amorce multiplexe allèle spécifique, d'un procédé basé sur des micropuces, et d'une hybridation dynamique allèle-spécifique.

7. Procédé selon la revendication 6, dans lequel la détermination est réalisée par amplification par PCR multiplexe utilisant des amorces spécifiques suivie de la détection.

8. Procédé selon l'une quelconque des revendications 1 à 7, dans lequel l'échantillon biologique est un échantillon de sang.

9. Jeu d'amorces pour réaliser le procédé selon l'une quelconque des revendications 2, 4 et 5, qui est constitué des amorces ayant les séquences : SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20 et SEQ ID NO : 21.

10. Utilisation d'un jeu d'amorces selon la revendication 9, pour le diagnostic de la démence à corps de Lewy.

11. Utilisation d'un procédé de génotypage du site polymorphe à la position 68974 dans le numéro d'accès NCBI NG_009031 (en d'autres termes, position 934 dans SEQ ID NO : 28), en combinaison avec une ou plusieurs modifications du gène BChE sélectionnées dans le groupe constitué de la région poly-thymine aux positions 4780 à 4786, du site polymorphe à la position 3687 ; du site polymorphe à la position 4206 ; et du site polymorphe à la position 4443, pour diagnostiquer la démence à corps de Lewy, lesdites positions faisant référence au numéro d'accès NCBI NG_009031 (en d'autres termes, positions 3687, 4206, 4443 et 4780 à 4786 respectivement dans SEQ ID NO : 1).

12. Utilisation selon la revendication 11, dans lequel le site polymorphe à la position 68974 est génotypé en combinaison avec la région poly-thymine aux positions 4780 à 4786.

13. Utilisation selon la revendication 11, dans laquelle le site polymorphe à la position 68974 est génotypé en combinaison avec la région poly-thymine aux positions 4780 à 4786, le site polymorphe à la position 3687 ; le site polymorphe à la position 4206 ; et le site polymorphe à la position 4443.


### SEQUENCE LISTING

<110> Fundació Institut d'Investigació en Ciències de la Salut
   Germans Trias i Pujol
Universitat Autònoma de Barcelona
<120> Genetic marker for the diagnosis of dementia with Lewy bodies
<130> P1589EP00
<160> 28
<170> PatentIn version 3.5
<210> 1
   <211> 5040
   <212> DNA
   <213> Homo sapiens
<220>
   <221> variation
   <222> (3687)..(3687)
   <223> single nucleotide polymorphism at position 3687 ofNCBI Accession
   Number NG_009031 (A3687G)
<220>
   <221> variation
   <222> (4206)..(4206)
   <223> single nucleotide polymorphism at position 4206 of NCBI Accession
   Number NG_009031 (A4206G)
<220>
   <221> variation
   <222> (4443)..(4443)
   <223> single nucleotide polymorphism at position 4443 of NCBI Accession
   Numbering_009031 (C4443T)
<220>
   <221> variation
   <222> (4780)..(4786)
   <223> polythymine region from position 4780 to 4786 of NCBI Accession
   Number NG_009031
<400> 1
   tccattttta tgttactatt tccaggaaaa atttaaaata gccttcatat acaataaaag 60
   caagttagat aaattatatt gtacttatac aaataattaa tacataggca tataaatgaa 120
   gttaaaaaaa tattcgatag cattattttg gaaaaactta agataggtta tatgaaagta 180
   ggctaccaaa gactatgtat gatgtatttc caaattagct tgaagcaaaa ggtacagttg 240
   tgcatgagaa taagcaatta aaaaaaagaa aaagcatata tatatatatg gtagcacaaa 300
   aatgtcctta gtatccaagc tccaggactt tgcctcttag tgtgtagttt gtgaactccc 360
   tctatcagct tcacagagga cagtagtata acgctgtcct caagtctact gaaaccaaag 420
   cccagggatg tggcctagaa atctggatgt ttaaatagca ctttgatgaa tttgataatg 480
   tgttactttg caatttttct gagagtgaga ctccaaagat agtcaagggt ttgggaagag 540
   taaatttaag ccaattcagt aaattaggtg ataaacaaag aagcttgctg tttttctctc 600
   tttttttatg ttttaaaaca atgagtttct ccttttttgc tcaagccctg aacaatttag 660
   ttcagaattt gttttcttac tttgattctg ctggctttgc caattccatg aaaatccact 720
   tggcaccaca tttccttaga agctcttgct ttttcttgat tttctttctg ttcttagagt 780
   ctaatattct agtgtctcac caaattgttg ttgtctcttt ccaaaactcc tttaaccaaa 840
   ctatctgtat cttcatgtaa aaggcaccac agacctccca tcactcactc tcccttaacc 900
   ccaaaatatc tatgtgttat cccttacaga tgtcacttcc tgcaaggcct tccatattag 960
   ttgctacaac atcactaaag aattttatta cctgtggctc tggacctact tctaatatct 1020
   ctggagatat ccaccttcaa acacctgcca catccagcta tgagggtcta gtacctgggc 1080
   tccagagaca tgttcaggtt agttctgatt ataagaacat ttctcttttt tgccagaatt 1140
   gttggaaagt tatctctatt caacagtctg tgtaggattt cagaagattg aatatacata 1200
   taaatatata tacatacaca catgtatata tacacacaca catatatgta tatgtacttt 1260
   gtccatgtcg tatttggtta ttccaattct gggaaaataa attaagaaaa taattaaaag 1320
   atgcactcaa atatcgatat acaaaatgcc tattgtaata atacataaaa aaagtcagaa 1380
   gctaacctcg gtatctgtat gatttggagc aagttactta aactctttgt ggctatattt 1440
   tctcaaatgt aaaataaggt taataatagt gcttatttta aaggtagtca tgagttttaa 1500
   atgaattcat gtattcaaag tggatagaat agtgctttca aataatattt attacctaaa 1560
   attattatta tcatcatata agtaacaaga gatacaatga accataatat gcccatatga 1620
   taatatatca cacagtaatc aaaatatttt aataggaaat taatatcata agataaaaag 1680
   caagaagaat gatgataagc catttgacta ccactatatt tatctatgta actataacca 1740
   catacacata ttttaaaaaa ttgattggaa tatacaaaat ggtcttttca ttctctgtga 1800
   tatgtaaaat gtagaaatat acttttctgg gctggatgca gtagctcaca cctataatcc 1860
   cagcactttg ggaggccgag gttggtggag cacctgaggt caggaattcg agaccagcct 1920
   ggccaacata gtgaaatcct gtgtctacta aaaatacaaa aattagccag atttggtggt 1980
   gggtgcctgt aatcccagct actcaggagg ctgaggcagg aaaattgctt gaacccagga 2040
   ggcagaggtt gcagtgagcc gagatcctgc cactgcactc cagcctgggc gacagagtga 2100
   gactccatct caggaaaaaa aaaaaaaaat ctatctatct atctatctat ctatctatct 2160
   atctatctat ctatctatct atacttatat atacttttct ttgttatcca tattcatttc 2220
   agagaatgca gtgctattgt aaaaaaaatg aataaaatta atataagttt agagcacttt 2280
   tttggcatca gtatgcctaa ggaaaactaa tgtataatac aggattaatg tttttcacac 2340
   catctcaaat cttactgaat gctgctatct agaaaacagt ggtaactgag ttacaatggg 2400
   accttaaaag caaaaaacca ctgtgactgg ctaactgaaa gtgatcatag agccaaaacc 2460
   ataatgattg tcctgcccaa gcttcagcat atatggttgc aaaatgtgcg cagtgttaat 2520
   gccgtgtcaa gtgtcttgtg gtatttcatc agtgtcgctt acaaggcata ctcaacattt 2580
   aacagcagga tcattaacca tccatgctgt gatgtaatgg aatagtgctt ccctactaac 2640
   cctgaaacaa gctcactgtg tgtgacctac atccttccgc tggatgagtg tgagaaaatg 2700
   atgacaccac agaagcaaag cagctgctgt aagttgagcc acaaggaaga aactctcaag 2760
   acaggaagaa taaatgcttt agaggtgcac taaaataatg aatgaactaa tgtggcatag 2820
   ctgtgaaatc atgcgaggaa aagaactaca gggacagtaa cagaaggtgg ttctttgaag 2880
   taaagacatt gagaagaaga aaaattgcca acttgtgtaa tcagttgatg ttgctagtta 2940
   gccagtttaa acatgcgtta aagataaaga tctgcttgaa accaattatt ttattgtttg 3000
   gcttcctgtt taaagatgaa aatgaaatat gtgtatatat gtgacatatt ttacctagca 3060
   gtgagtagat gcaggaagca tgtaaaatgc tttgctgaat tcactaacgt cacaaaatat 3120
   tccttaataa ttgccgcgtt tgataggctg accttatgct agcacaggaa acaatgcagt 3180
   ggacttgggt tggagagaag aattttgaca tttggatttt taaaaatcat gtatattgaa 3240
   cccatgaact ctcatgtgga acatcagatt tactatattt taaacagaaa tgccatcact 3300
   ataaaaacca ttgcaaattt gtcaatatac ttccatttaa aaagcctttt cctgctttta 3360
   ttgatttatt gattaatttt tttttgagac aggatctcac tctgtcaccc acgatgaagt 3420
   gcagtggcac aatcgtggct tgcagcctcg acctcctagg ctcaggtgat actcttacct 3480
   cagctacctc ccaagtaact gggaatgtag gcttgcacca ccatgtctga cccattttct 3540
   tgcagaaatt gagttttgct atgttgtcca gacttgtctt gaactcccag actgaagcaa 3600
   tccgcccact taggcctcct aaaatgttgg gattacagtc atgaaccact gtgctccacc 3660
   tcctttttct ccttttcaga tagattatgt attctgtacc ttttgtgtaa ctaacttgtt 3720
   attacataaa ctatattcta aatacattat aaattattgt gatcatgata tttatcattg 3780
   aattaacact gcctaagtag caattttcac agaaaagata attttaaaag accaaaatgg 3840
   ttattctctt ggaagcagtt gacataaaat aacagaataa tacagattaa aattctgttt 3900
   tccaacagct actagtggta agaaatatag taagcttctg atttgtagaa accttagctt 3960
   tctcatctga tgaggtggtt aataatatta actctcatat ttgctgaaat aatataaaat 4020
   atttaatata aattgtcaat tataatgact ggcataaggt agaattacaa caaatgctaa 4080
   ttatatttgc ttgtttgaaa atttaacact ctattaaaag gcaaaaaagt gctcacctgc 4140
   aaatattaat acatgccttc tgtttagtcc atagaaagcc cacaagtaag agattcaaaa 4200
   gtgaaagcta tcacttacag aagattaaga atatattgca ttgagaagat agggattaag 4260
   gagtttatca aaggtttttt aaaaaattat tttcctgaac atcatttcaa gaaagataag 4320
   cgccaaagac ataaatatat tctccagagc atgtgcactg caagttgaca aatgtgtctc 4380
   ttttatagag tttggtgaag gttattcatt ttatttttcc tgtcttgacc agaaaattgt 4440
   ggcttctcat catgtatgac tgcctgcagg gtcttctgaa taggaaatta ccaatgctga 4500
   aatttgtatg acttaaataa gtgttgaagt gtgggggttg aaaggagata catatcagag 4560
   acatccattc agttaataga actaagtttt tgatttgcat agcttatatc tagagcattg 4620
   tgtttcttat tttctatatt actatcttct ctaactatat aattagcaca gattgaagct 4680
   ataatggatt tcaataatag cactacttta gaatgatgta tacgtcttta aatcttaaga 4740
   caagcatagg aaatatgtat attcttaaaa aaaggaaaat ttttttaaag tttagagtta 4800
   aaagaaacat gcattttctg ttacatttca gattatttta gttaaagaat cttctatgct 4860
   ttttttcttc attcttagaa gactacctgc aattgtaaag catgaatcct gtaatacttc 4920
   tgtaaatgaa tcactattgc atttatacca ttagcctctg aacagatttc aagttgctgc 4980
   tgccaactct cgcgagcttt gtcagtaaca gttgattgtt acattcagta acactgaatg 5040
<210> 2
   <211> 20
   <212> DNA
   <213> Artificial Sequence
<220>
   <223> Primer for BChE promoter sequencing: BChEpromlUA
<400> 2
   tgataggctg accgtatgct 20
<210> 3
   <211> 20
   <212> DNA
   <213> Artificial Sequence
<220>
   <223> Primer for BChE promoter sequencing: BChEprom1L
<400> 3
   acctcatcag atgagaaagc 20
<210> 4
   <211> 21
   <212> DNA
   <213> Artificial Sequence
<220>
   <223> Primer for BChE promoter sequencing: BChEprom2UA
<400> 4
   tctcttggaa gcagttgaca t 21
<210> 5
   <211> 22
   <212> DNA
   <213> Artificial Sequence
<220>
   <223> Primer for BChE promoter sequencing: BChEpromS6
<400> 5
   ccattatagc ttcaatctgt gc 22
<210> 6
   <211> 24
   <212> DNA
   <213> Artificial Sequence
<220>
   <223> Primer for BChE promoter sequencing: BChEprom2UB
<400> 6
   agatacatat cagagacatc catt 24
<210> 7
   <211> 20
   <212> DNA
   <213> Artificial Sequence
<220>
   <223> Primer for BChE promoter sequencing: BChEprom2L
<400> 7
   gaagagatca ctctcatccc 20
<210> 8
   <211> 23
   <212> DNA
   <213> Artificial Sequence
<220>
   <223> Primer for genotyping A-1314G: BChE-1314U
<400> 8
   tcttgaactc ccagactgaa gca 23
<210> 9
   <211> 23
   <212> DNA
   <213> Artificial Sequence
<220>
   <223> Primer for genotyping A-1314G: BChE -1314G
<400> 9
   tacacaaaag gtacagaata cac 23
<210> 10
   <211> 43
   <212> DNA
   <213> Artificial Sequence
<220>
   <223> Primer for genotyping A-1314G: BChE -1314A
<400> 10
   ttatgtaata acaagttagt tacacaaaag gtacagaata cat 43
<210> 11
   <211> 23
   <212> DNA
   <213> Artificial Sequence
<220>
   <223> Primer for genotyping A-795G: BChE -795U
<400> 11
   aagtgctcca cctgcaaata tta 23
<210> 12
   <211> 22
   <212> DNA
   <213> Artificial Sequence
<220>
   <223> Primer for genotyping A-795G: BChE -795G
<400> 12
   taatcttctg taagtgatag cc 22
<210> 13
   <211> 42
   <212> DNA
   <213> Artificial Sequence
<220>
   <223> Primer for genotyping A-795G: BChE -795A
<400> 13
   ttctcaatgc aatatattct taatcttctg taagtgatag ct 42
<210> 14
   <211> 22
   <212> DNA
   <213> Artificial Sequence
<220>
   <223> Primer for genotyping C-558T: BChE -558L
<400> 14
   tgtctctgat atgtatctcc tt 22
<210> 15
   <211> 21
   <212> DNA
   <213> Artificial Sequence
<220>
   <223> Primer for genotyping C-558T: BChE -558CS
<400> 15
   tcttgaccag aaaattgtgg c 21
<210> 16
   <211> 42
   <212> DNA
   <213> Artificial Sequence
<220>
   <223> Primer for genotyping C-558T: BChE -558TL
<400> 16
   tattcatttt atttttcctg tcttgaccag aaaatttgtg gt 42
<210> 17
   <211> 27
   <212> DNA
   <213> Artificial Sequence
<220>
   <223> Primer for genotyping BchE-K: BchE-4U
<400> 17
   ctgtactgtg tagttagaga aattggc 27
<210> 18
   <211> 30
   <212> DNA
   <213> Artificial Sequence
<220>
   <223> Primer for genotyping BchE-K: BchE-K
<400> 18
   atggaatcct gctttccact cccattccgt 30
<210> 19
   <211> 50
   <212> DNA
   <213> Artificial Sequence
<220>
   <223> Primer for genotyping BchE-K: BchE-W
<400> 19
   atcatgtaat tgttccagcg taggaatcct gctttccact cccattctcc 50
<210> 20
   <211> 26
   <212> DNA
   <213> Artificial Sequence
<220>
   <223> Primer for genotyping polyT: BChEpolyT-U
<220>
   <221> modified_base
   <222> (1)..(1)
   <223> FAM (6-carboxyfluorescein)
<400> 20
   tcaataatag cactacttta gaatga 26
<210> 21
   <211> 23
   <212> DNA
   <213> Artificial Sequence
<220>
   <223> Primer for genotyping polyT: BChEpolyT-L
<400> 21
   aggtagtctt ctaagaatga aga 23
<210> 22
   <211> 22
   <212> DNA
   <213> Artificial Sequence
<220>
   <223> Primer BChE 2U for real time PCR
<400> 22
   gagtagatcc atagtgaaac gg 22
<210> 23
   <211> 20
   <212> DNA
   <213> Artificial Sequence
<220>
   <223> Primer BChE 6LRNA for real time PCR
<400> 23
   cagcgatgga atcctgcttt 20
<210> 24
   <211> 20
   <212> DNA
   <213> Artificial Sequence
<220>
   <223> Primer beta-actin U2 for real time PCR
<400> 24
   tctacaatga gctgcgtgtg 20
<210> 25
   <211> 20
   <212> DNA
   <213> Artificial Sequence
<220>
   <223> Primer beta-actin L3 for real time PCR
<400> 25
   tagatgggca cagtgtgggt 20
<210> 26
   <211> 20
   <212> DNA
   <213> Artificial Sequence
<220>
   <223> Primer GUS-U1 for real time PCT
<400> 26
   atgtggttgg agagctcatt 20
<210> 27
   <211> 20
   <212> DNA
   <213> Artificial Sequence
<220>
   <223> Primer GUS-L2 for real time PCR
<400> 27
   tgtctctgcc gagtgaagat 20
<210> 28
   <211> 1980
   <212> DNA
   <213> Homo sapiens
<220>
   <221> variation
   <222> (934)..(934) <223> single nucleotide polymorphism corresponding to position 68974 of the NCBI Accession Number NG_009031 (G68974A). It is the K variant polymorphism. This SEQ ID NO: 28 represents the region from position 68041 to 70020 of this Accession Number.
<400> 28
   ggaggcagag gttgcggtga gccaagatgg cgccattgca ctccagcctg ggtggcaaga 60
   gtgaactctg tctcaaaaaa aaataaataa ataaaataaa acattgtatt ataataaggg 120
   taaaatctat gtggaatgat aattaaaaag ttatccttat gcattaataa atacagtatg 180
   acctttgtat ttgcgggtta cacatccatg gatttaacca accacgagtc aaaaacattt 240
   gataaataaa tacatctata ctaaacatat acagaccttt ttttcttatc attattcctt 300
   aaacaatata atataataaa catttacaca gcactttcat tatattaggt atgaattatc 360
   tagggatgtg catggcttat gtgaaaatgt tatgctattt tacattaggg acttgagtat 420
   gagaggattt tggtatccct gagaggtcct agaaccaact ccccacaaat actaaggatg 480
   actgtagtca tatataattt tcatcagaaa taattatctg ttatataact atatatgtaa 540
   atgagaccat taaatgaaat ttgtgttatt ttaaagtggg tcaagaaaag agcataatat 600
   ctacatatcc tcttgaaaac taaagaaaat atatgtaatg taattataca taatacaata 660
   tataaactca ttttccattt tatatgttat acgttatata aattatatat acattatttg 720
   cattttcaca taatctgtaa agattttatt aaaatctctt ttcaggcaaa gcgagctaat 780
   aacaaataat aaagaataaa taaagaaaat aatgctgtac tgtgtagtta gagaaaatgg 840
   cttttgtatt cgaaattatt tttcagttaa tgaaacagat aaaaattttg attaatacaa 900
   cttattccat attttacagg aaatattgat gaagcagaat gggagtggaa agcaggattc 960
   catcgctgga acaattacat gatggactgg aaaaatcaat ttaacgatta cactagcaag 1020
   aaagaaagtt gtgtgggtct ctaattaata gatttaccct ttatagaaca tattttcctt 1080
   tagatcaagg caaaaatatc aggagctttt ttacacacct actaaaaaag ttattatgta 1140
   gctgaaacaa aaatgccaga aggataatat tgattcctca catctttaac ttagtatttt 1200
   acctagcatt tcaaaaccca aatggctaga acgtgtttaa ttaaatttca caatataaag 1260
   ttctacagtt aattatgtgc atattaaaac aatggcctgg ttcaatttct ttctttcctt 1320
   aataaattta agtttttccc cccaaaatta tcagtgctct gcttttagtc acgtgtattt 1380
   tcattaccac tcgtaaaaag gtatcttttt taaatgaatt aaatattgaa acactgtaca 1440
   ccatagttta caatattatg tttcctaatt aaaataagaa ttgaatgtca atatgagata 1500
   ttaaaataag cacagaaaat cattggtctc tttgtttttt atataaaggc aagaaagaat 1560
   cttaaaatga gaggctcagc cactttaaaa caccttataa tagaaacaga atgctgctac 1620
   caagaaacaa aatataaaat aatgtaatta tttatgtaat gccttcaaat tatattcttc 1680
   atttgactct tgctctccct cacctcctct atcctcaaat caccacaaac atgtacatat 1740
   gtgaattacc tttccgtaat tcaaaaattt ttaaaaagtg ggtgcataga agtctaaagt 1800
   taggttgtgt accatctggg acagggcaac ccttgcctgg acccagaaat attaacatat 1860
   taataggaaa attgttcaat tccagaagag aaatggttgt actttctgta gacaaactgt 1920
   cttatttttg caattaaaaa aattacagag ctgcatttat tagcatgctt ggcaagggag 1980